# EUROPEAN PATENT SPECIFICATION

(11) **EP 0 567 452 B1**
(45) Date of publication and mention of the grant of the patent: **15.05.1996**
(21) Application number: 91902000.8
(22) Date of filing: 18.12.1990
(51) Int. Cl.: A61M 1/16

(54) **DIALYSATE PRODUCTION SYSTEM WITH DIALYSATE PELLETS**
DIALYSAT-PRODUKTIONSSYSTEM MIT PILLEN
SYSTEME DE PRODUCTION DE DIALYSAT A L'AIDE DE PASTILLES DE DIALYSAT

(43) Date of publication of application: 03.11.1993
(73) Proprietor: THE BOARD OF REGENTS OF THE UNIVERSITY OF WASHINGTON, Seattle WA 98105 (US)
(72) Inventor: AHMAD, Suhail, Seattle, WA 98125 (US); COLE, James, J., Arlington, WA 98223 (US); JENSEN, William, Seattle, WA 98125 (US)
(74) Representative: Grünecker, Kinkeldey, Stockmair & Schwanhäusser Anwaltssozietät
(86) International application number: US9007480
(87) International publication number: WO9211046

(56) References cited:
- EP-A- 0 034 916
- EP-A- 0 399 918
- FR-A- 2 569 560
- US-A- 4 734 198

## Description

The present invention relates to hemodialysis systems, and more particularly, to an improved system for supplying dialysates and dry dialysate compositions.

Hemodialysis treatment is employed as a therapeutic measure when a patient's kidneys no longer perform their blood purifying function because of disease or traumatic removal. Kidney failure results in the accumulation of toxic waste in the patient's blood and eventual death from uremic poisoning, unless the waste material is removed by some artificial means. In hemodialysis of the type to which the present invention relates, the patient's blood is circulated from the patient in a closed blood circuit by a pump to one side of a membrane contained within a hemodialyzer (i.e., artificial kidney). The membrane has pores of microscopic size through which waste products from the blood pass. The pores are, however, too small to permit blood cells and proteins to leave the body. A dialysis fluid (dialysate) is circulated on the other side of the hemodialyzer membrane to remove the waste products. The dialyzed blood is returned to the patient.

Commonly the dialysate for hemodialysis systems is supplied as a liquid concentrate in containers from which it is blended and diluted with sterile water by the use of proportional pumping systems.

EP-A-0 399 918 discloses a two component dry dialysate composition, i.e. a first powdery composition comprising (a) solid electrolytes for dialysis and a liquid acid, (b) solid electrolytes for dialysis, glucose and a liquid acid, or (c) solid inorganic salts for dialysis, glucose, sodium acetate and acetic acid, and a second composition comprising (a) sodium hydrogen carbonate and glucose, (b) sodium carbonate or (c) sodium hydrogen carbonate and sodium acetate.

It is the object underlying the present invention to provide novel dry dialysate compositions and an improved dialysate production system.

According to the present invention this object is achieved with
(a) a dry dialysate composition comprising a pellet with a plurality of separated layers of an acid, bicarbonate and a salt, wherein the acid will dissolve first in an aqueous solution and the bicarbonate will dissolve after solution of the acid;
(b) a dry dialysate composition in a pellet or tablet form comprising an acid, a base and a salt, wherein the acid is selected from the group consisting of citric acid, lactic acid, ascorbic acid, acetic acid and combinations thereof and wherein the base is selected from the group consisting of bicarbonate, carbonate, lactate, citrate and combinations thereof;
and
(c) a dry dialysate composition in a pellet or tablet form comprising an acid, a base and a salt wherein the acid is selected from the group consisting of citric acid, ascorbic acid and combinations thereof and wherein the base is selected from the group consisting of bicarbonate, carbonate, lactate, citrate and combinations thereof.

Furthermore, the present invention provides a dialysate production system comprising:
a plurality of dry dialysate compositions according to the present invention;
a mixing tank;
a gating device arranged and adapted to control the addition of dry dialysate pellets to said mixing tank;
a water source;
a means for circulating a fixed volume of water from said water source to the mixing tank to dissolve a dry dialysate pellet therein to form dialysate in the mixing tank;
and a circulating means for circulating said dialysate from the mixing tank to a hemodialyzer.

Preferred embodiments of the present invention are set forth in the dependent claims.

The present invention provides an on-site dialysate production system for supplying dialysate directly to a hemodialysis system by utilizing dry chemical pellets or tablets, wherein the pellet or tablet contains an acid or acids, a base or bases, and salts, with the proviso that the acid component be separated from the base component. The pellets are added to mixing chambers containing treated water to form the dialysate. The mixed dialysate from the chambers flows into the dialysate circuit through the hemodialyzer and/or hemofilter. Preferably, the acid component is citric acid, and this forms an effervescence upon contact with water and other chemicals to facilitate the solution of the dry chemical into the dialysate and maintains a pH below 7.4. Moreover, the more acid pH prevents calcium carbonate from forming an insoluble precipitate in the aqueous solution.

Figure 1 is a schematic of the main components in a traditional hemodialysis system.

Figure 2 is a schematic of a dialysate production system embodying the present invention.

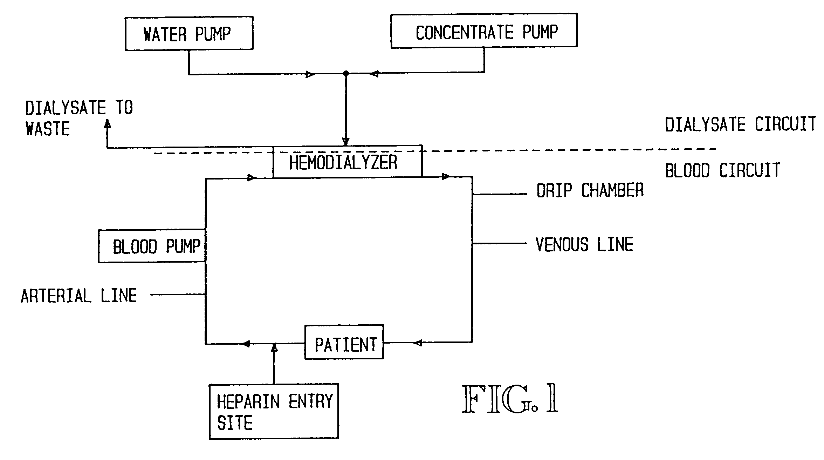

Referring to Figure 1, an "arterial" line runs from the patient to a blood pump and then to one side of the membrane in the hemodialyzer. The blood then flows to a drip chamber in the "venous" line and back into the patient. This forms the blood circuit. Conventionally, the dialysate circuit has been formed by mixing liquid dialysate concentrate with sterile water and passing the resulting dialysate on the other side of the hemodialyzer membrane and then continuing out to waste.

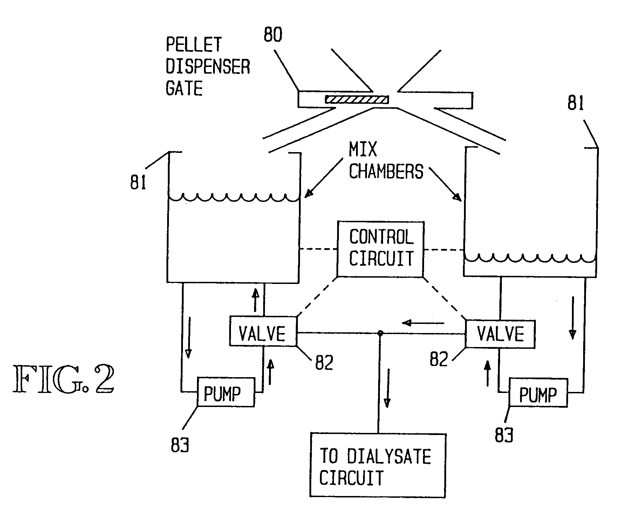

Referring to Figure 2 in the dialysate production system of this invention, dry chemical pellets or tablets in a hopper or magazine are dropped, or otherwise fed, into a pellet dispenser gate (80) and then added to one of two mixing chambers (81) containing a predetermined amount of sterile water. Preferably, a pump (83) circulates the water in the mix chamber to dissolve the pellet, and form a dialysate solution. Citric acid in the pellet regulates the pH of the dialysate to pH 7.4 or below to prevent calcium carbonate precipitate from forming. A valve (82) controls the addition of the dialysate in the mix chamber to the dialysate circuit. This system eliminates the need for use of a concentrate proportioning pump in prior art systems.

In accordance with the present invention, dry chemicals are formed into the pellets in premeasured amounts. Each pellet contains an acid, base, and salt in dry form. Preferably, the acid is citric acid and is separated from the base and salt. Preferably, the pellets are formed and stored under low humidity conditions. The pelletized dry chemicals are capable of forming dialysates with either acetate-based or bicarbonate-based dialysates without equipment conversion. Preferably, the salt forms a barrier layer between the acid and the base in the pellet.

The dry chemicals suitable for use in the pellets include salts comprising an anion and a cation, wherein the anions are selected from the group consisting of bicarbonate, citrate, chloride, acetate, lactate, and combinations thereof; and wherein the cations are selected from the group consisting of sodium, potassium, magnesium, calcium, and combinations thereof. Additional organic dry chemicals suitable for use as salts include dextrose and urea. Useful acids include citric acid, lactic acid, ascorbic acid and acetic acid. Typical bases include bicarbonate, carbonate, lactate and citrate. Preferably, sodium, potassium, calcium, and magnesium are the cations. A suitable dry dialysate composition in pellet form that can be mixed with 1 l of water to form 1 l of dialysate comprises from about 130 to about 150 mEq Na, from 0 to about 4.0 mEq of K, from about 2.0 to 3.5 of mEq Ca, from 0 to about 1.5 mEq Mg, from about 25 to about 45 mEq bicarbonate, from 0 to about 2 g/L glucose, and from about 90 to about 120 mEq chloride ion. Acetate or lactate can be substituted for bicarbonate at the same concentration range. Preferably, citric acid is used at a concentration from about 2 to 12 mEq to maintain an acid pH of the dialysate.

Each mix chamber 81 can contain, for example, from about 2 to about 10 l of dialysate. Each dialysate chamber volume can be prepared by mixing an appropriate volume of water with a single pellet. The valves 83 located in the pump circuit can switch a mix chamber into a dialysate reservoir to pump dialysate through the dialysate circuit to the hemodialyzer and out to waste. The second mix chamber can be preparing the next reservoir of dialysate for use when the first mix chamber becomes empty. Hence, preferably there are at least two mixing chambers 81.

The use of citric acid in conjunction with conventional dialysate chemicals produces a mixture which will dissolve quickly and completely in the time required by the system. The resulting citrate load is well tolerated, and causes no disturbance of the blood calcium level. Construction of the pellet, such that the more acid components dissolve first, maintains the pH of the solution below the level of 7.40 at all times. This chemical environment prevents the formation of insoluble precipitates, especially calcium salts.

The pellets can be loaded in prescribed order in a suitable pellet dispenser means controlled by the pellet dispenser gate 80 to change the ion gradient of the dialysate during the treatment process to better suit the individual patient's treatment needs.

It is possible to attach a bar code to the pellet and an optical scanner in the means for adding pellets to the mixing chambers to ensure proper gradient formation and to allow the mixing system to adjust monitoring according to pellet composition. The pellets can be preloaded in magazines or casettes.

As previously indicated, the utilization of discrete tablets or pellets makes it possible to easily change the chemical makeup of the dialysate during treatment in accordance with changing requirements of the individual patient. For example, Raja et al., "Role of Varying Dialysate Sodium and Bicarbonate in the Improvement of Dialysis Vascular Stability," Prog. Art. Organs, Nose et al. (eds.), ISAO Press, Cleveland, 1985, pp. 237-39 [Raja et al. I], and Raja et al., "Sequential Changes in Dialysate Sodium (DNa) During Hemodialysis," Trans. Am. Soc. Artif. Intern. Organs 29:649-651, 1983 [Raja et al. II] describe several schemes to vary dialysate ion concentrations during treatment. The ability to introduce, in prescribed order, pellets with different chemical makeup into the mixing chambers makes possible the timed adjustment in individual dialysate ion concentrations during dialysis treatment in accordance with the prescription of the managing physician.

For example, the dialysate sodium concentration can be progressively changed from 150 to 135 mEq/L in decrements of 1 or 2 mEq/L during the course of treatment. At the same time, the bicarbonate concentration might be altered from 20 to 35 mEq/L in 5 mEq/L increments during the first 3 hours of the procedure. The dialysate chemical composition can be flexibly changed every few minutes, as each new pellet is introduced, to produce optimal treatment results according to the defined needs of the individual patient. It will be appreciated that the system can be automated and programmed to control the feeding of the pellets and delivery of the dialysate to the dialysis circuit.

Another example of the benefit of being able to vary the dialysate ion concentration during treatment is to control the rate of osmolar change during dialysis. Several treatment-related symptoms during dialysis have been shown to be related to osmolar decline, and the reduction or blunting in this decline can also reduce treatment symptoms, thus improving the quality of dialysis. One way to achieve this goal is to use sodium modeling. The sodium concentration in the dialysate is increased in the early phase of dialysis and then is slowly reduced to lower concentrations, thus blunting the rate of decline of blood osmolarity. Sodium modeling can only be accomplished, at present, with additional equipment added to a basic dialysis system, and then the procedure is nonselective, altering both sodium and other ions proportionally. The present invention achieves sodium modeling by loading dry dialysate pellets with higher sodium concentrations for the early part of dialysis treatment and then gradually using pellets with lower sodium concentrations throughout the remainder of the treatment. Similarly, other osmolar agents, for example urea, can be added.

In present dialysis systems, changing the sodium concentration also proportionally alters the concentrations of other constituents, such as calcium and magnesium. Because individual pellets can be introduced at frequent intervals with the inventive system, the concentrations of all ionic species, except those whose change is desired, can be held constant.

It will be appreciated that, although the invention has been described with respect to dialysate for hemodialysis, it is also applicable to supplying dialysate for peritoneal dialysis, in which case greater quantities of glucose can be used, and the dialysate circuit connects to the patient rather than to the hemodialyzer.

## Claims

1. A dry dialysate composition comprising a pellet with a plurality of separated layers of an acid, bicarbonate and a salt, wherein the acid will dissolve first in an aqueous solution and the bicarbonate will dissolve after solution of the acid.

2. The dry dialysate composition of claim 1 wherein the acid is citric acid.

3. The dry dialysate composition of claim 1 wherein, upon dissolving in water, the pH remains below 7.4.

4. A dry dialysate composition in a pellet or tablet form comprising an acid, a base and a salt, wherein the acid is selected from the group consisting of citric acid, lactic acid, ascorbic acid, acetic acid and combinations thereof and wherein the base is selected from the group consisting of bicarbonate, carbonate, lactate, citrate and combinations thereof.

5. The dry dialysate composition of claim 4 comprising:
from about 130 to about 150 mEq/L of sodium ion;
from about 0 to about 4.0 mEq/L of potassium ion;
from about 2.0 to about 3.5 mEq/L of calcium ion;
from about 0 to about 1.5 mEq/L of magnesium ion;
from about 25 to about 45 mEq/L of bicarbonate ion,
acetate, lactate or combinations thereof;
from about 0 to about 2.0% glucose;
from about 90 to about 120 mEq/L of chloride ion;
and
from about 2 to about 12 mEq/L of citric acid;
and forming a dialysate upon mixing with water.

6. A dry dialysate composition in a pellet or tablet form comprising an acid, a base and a salt wherein the acid is selected from the group consisting of citric acid, ascorbic acid and combinations thereof and wherein the base is selected from the group consisting of bicarbonate, carbonate, lactate, citrate and combinations thereof.

7. A dialysate production system comprising:
a plurality of dry dialysate compositions according to any one of claims 1 to 6;
a mixing tank;
a gating device arranged and adapted to control the addition of dry dialysate pellets to said mixing tank;
a water source;
a means for circulating a fixed volume of water from said water source to the mixing tank to dissolve a dry dialysate pellet therein to form dialysate in the mixing tank;
and a circulating means for circulating said dialysate from the mixing tank to a hemodialyzer.

8. The dialysate production system of claim 7 wherein there is a second mixing tank operatively associates with said gating device, water source and circulating means whereby dialysate may be alternately circulated from said tanks.

9. The dialysate production system of claim 7 wherein the salt comprises an anion and a cation.

10. The dialysate production system of claim 9 wherein the anion is selected from the group consisting of bicarbonate, lactate, citrate, chloride, acetate and combinations thereof.

11. The dialysate production system of claim 9 wherein the cation is selected from the group consisting of sodium, potassium, magnesium, calcium and combinations thereof.

## Patentansprüche

1. Trockendialysatzusammensetzung, umfassend ein Pellet mit einer Vielzahl von getrennten Schichten einer Säure, eines Bicarbonats und eines Salzes, worin sich die Säure zuerst in einer wässerigen Lösung löst und das Bicarbonat sich nach dem Lösen der Säure löst.

2. Trockendialysatzusammensetzung nach Anspruch 1, worin die Säure Zitronensäure ist.

3. Trockendialysatzusammensetzung nach Anspruch 1, worin der pH-Wert nach dem bei Lösen in Wasser unter 7,4 bleibt.

4. Trockendialysatzusammensetzung in Pellet- oder Tablettenform, umfassend eine Säure, eine Base und ein Salz, worin die Säure ausgewählt ist aus der Gruppe, bestehend aus Zitronensäure, Milchsäure, Ascorbinsäure, Essigsäure und deren Kombinationen und worin die Base ausgewählt ist aus der Gruppe, bestehend aus Bicarbonat, Carbonat, Lactat, Citrat und deren Kombinationen.

5. Trockendialysatzusammensetzung nach Anspruch 4, umfassend:
etwa 130 bis etwa 150 mEq/L Natriumion,
etwa 0 bis etwa 4,0 mEq/L Kaliumion,
etwa 2,0 bis etwa 3,5 mEq/L Calciumion,
etwa 0 bis etwa 1,5 mEq/L Magnesiumion,
etwa 25 bis etwa 45 mEq/L Bicarbonation,
Acetat, Lactat oder deren Kombinationen, etwa 0 bis etwa 2,0% Glucose,
etwa 90 bis etwa 120 mEq/L Chloridion, und
etwa 2 bis etwa 12 mEq/L Zitronensäure, und
das Bilden eines Dialysats bei Mischen mit Wasser.

6. Trockendialysatzusammensetzung in Pellet- oder Tablettenform, umfassend eine Säure, eine Base und ein Salz, worin die Säure ausgewählt ist aus der Gruppe, bestehend aus Zitronensäure, Ascorbinsäure und deren Kombinationen und worin die Base ausgewählt ist aus der Gruppe, bestehend aus Bicarbonat, Carbonat, Lactat, Citrat und deren Kombinationen.

7. Dialysaterzeugungssystem, umfassend:
eine Vielzahl von Trockendialysatzusammensetzungen nach einem der Ansprüche 1 bis 6,
einen Mischbehälter,
eine Einlaßvorrichtung, die zur Steuerung der Zugabe von Trockendialysatpellets zu dem Mischbehälter angeordnet und eingerichtet ist,
eine Wasserquelle,
eine Einrichtung, um ein feststehendes Volumen an Wasser aus der Wasserquelle dem Mischbehälter im Kreislauf zuzuführen, um ein Trockendialysatpellet darin zu lösen, um in dem Mischbehälter ein Dialysat zu bilden,
und eine Kreislaufführungseinrichtung, um das Dialysat aus dem Mischbehälter einem Hämodialysator im Kreislauf zuzuführen.

8. Dialysaterzeugungssystem nach Anspruch 7, worin es einen zweiten Mischbehälter gibt, der funktionsmäßig der Einlaßvorrichtung, der Wasserquelle und der Kreislaufführungseinrichtung zugeordnet ist, wodurch das Dialysat alternativ von diesen Behältern aus im Kreislauf geführt werden kann.

9. Dialysaterzeugungssystem nach Anspruch 7, worin das Salz ein Anion und ein Kation umfaßt.

10. Dialysaterzeugungssystem nach Anspruch 9, worin das Anion ausgewählt ist aus der Gruppe, bestehend aus Bicarbonat, Lactat, Citrat, Chlorid, Acetat und deren Kombinationen.

11. Dialysaterzeugungssystem nach Anspruch 9, worin das Kation ausgewählt ist aus der Gruppe, bestehend aus Natrium, Kalium, Magnesium, Calcium und deren Kombinationen.

## Revendications

1. Composition de dialysat sec comprenant une pastille munie d'une pluralité de couches séparées d'un acide, d'un bicarbonate et d'un sel, dans laquelle l'acide se dissout en premier dans une solution aqueuse et le bicarbonate se dissout après dissolution de l'acide.

2. Composition de dialysat sec selon la revendication 1, dans laquelle l'acide est de l'acide citrique.

3. Composition de dialysat sec selon la revendication 1, dans laquelle, après dissolution dans l'eau, le pH reste inférieur à 7,4.

4. Composition de dialysat sec sous forme de pastille ou tablette comprenant un acide, une base et un sel, dans laquelle l'acide est choisi dans le groupe comprenant l'acide citrique, l'acide lactique, l'acide ascorbique, l'acide acétique et des combinaisons de ceux-ci et dans laquelle la base est choisie dans le groupe comprenant les bicarbonates, les carbonates, les lactates, les citrates et des combinaisons de ceux-ci.

5. Composition de dialysat sec selon la revendication 4, comprenant :
d'environ 130 à environ 150 mEq/L d'ions sodium ;
d'environ 0 à environ 4,0 de mEq/L d'ions potassium ;
d'environ 2,0 à environ 3,5 mEq/L d'ions calcium ;
d'environ 0 à environ 1,5 mEq/L d'ions magnésium ;
d'environ 25 à environ 45 mEq/L d'ions bicarbonate,
acétate, lactate ou leurs combinaisons ;
d'environ 0 à environ 2,0 % de glucose ;
d'environ 90 à environ 120 mEq/L d'ions chlorure, et
d'environ 2 à environ 12 mEq/L d'acide citrique ;
et formant un dialysat par mélange avec de l'eau.

6. Composition de dialysat sec sous forme de pastille ou de tablette comprenant un acide, une base et un sel, dans laquelle l'acide est choisi dans le groupe comprenant l'acide citrique, l'acide ascorbique et leurs combinaisons et dans laquelle la base est choisie dans le groupe comprenant les bicarbonates, les carbonates, les lactates, les citrates et leurs combinaisons.

7. Système de production d'un dialysat comprenant :
une pluralité de compositions de dialysat sec selon l'une quelconque des revendications 1 à 6,
un réservoir de mélange,
un dispositif de porte d'entrée agencé et adapté pour contrôler l'addition de pastilles de dialysat sec dans le réservoir de mélange,
une source d'eau,
un moyen pour faire circuler un volume fixe d'eau de la source d'eau au réservoir de mélange pour dissoudre une pastille de dialysat sec dans celui-ci pour former un dialysat dans le réservoir de mélange, et
un moyen de circulation pour faire circuler le dialysat du réservoir de mélange vers un hémodialyseur.

8. Système de production de dialysat selon la revendication 7, dans lequel il existe un second réservoir de mélange opérativement associé au dispositif de porte d'entrée, à la source d'eau et au moyen de circulation, d'où il résulte que le dialysat peut être amené à circuler de façon alternée à partir des réservoirs.

9. Système de production de dialysat selon la revendication 7, dans lequel le sel comprend un anion et un cation.

10. Système de production de dialysat selon la revendication 9, dans lequel l'anion est choisi dans le groupe comprenant les bicarbonates, les lactates, les citrates, les chlorures, les acétates et leurs combinaisons.

11. Système de production de dialysat selon la revendication 9, dans lequel le cation est choisi dans le groupe comprenant le sodium, le potassium, le magnésium, le calcium et leurs combinaisons.
